# EUROPEAN PATENT APPLICATION

(11) **EP 4 818 169 A1**
(43) Date of publication of application: **07.10.2026**
(21) Application number: 25760891.9
(22) Date of filing: 25.02.2025
(51) Int. Cl.: C07D 487/04, A61K 31/395, A61K 31/519, A61P 35/00

(54) **CRYSTAL FORM OF COMPOUND SALT, PREPARATION METHOD THEREFOR AND USE THEREOF**

(30) Priority: 26.02.2024 CN 202410211544
(71) Applicant: Apeiron Therapeutics (Hong Kong) Ltd., Central, Hong Kong 999077 (HK)
(72) Inventor: ZHOU, Yanfeng, Shanghai 201210 (CN)
(74) Representative: Bayramoglu et al.
(86) International application number: PCT/CN2025/078953
(87) International publication number: WO 2025/180350

(57) **Abstract**

Provided are a crystal forms of a (S)-4-amino-N-methyl-N-(6-(trifluoromethyl)-2,3-dihydrobenzofuran-3-yl)imidazo[1,5-a]quinoxaline-8-carboxamide (hereinafter referred to as "Compound I") salt, a preparation method therefor, a pharmaceutical composition containing the compound I salt, and a use of the compound I salt in preparation of a PRMT5 inhibitor drug.

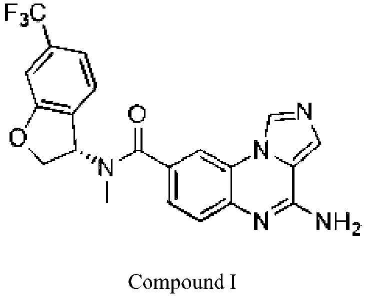

## Description

### Priority Declaration

The present disclosure claims priority to Chinese Patent Application No. 202410211544.6, filed on February 26, 2024. The full disclosure of the above-referenced Chinese patent application is incorporated herein by reference.

### Technical Field

The present disclosure relates to the field of crystal chemistry. Specifically, it relates to a pharmaceutically acceptable salt of (S)-4-amino-N-methyl-N-(6-(trifluoromethyl)-2,3-dihydrobenzofuran-3-yl)imidazo[1,5-a]quinoxaline-8-carboxamide and its crystalline form.

### Background Art

(S)-4-amino-N-methyl-N-(6-(trifluoromethyl)-2,3-dihydrobenzofuran-3-yl)imidazo[1,5-a]quinoxaline-8-carboxamide has the structure of formula (I), which is referred to herein as Compound I:

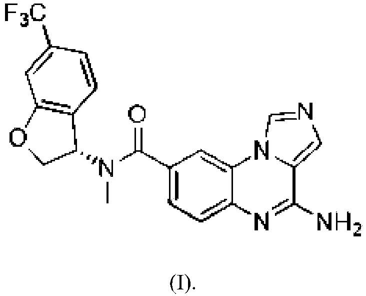

In the research and development of small-molecule drugs, drug polymorphism is a common phenomenon and an important factor affecting drug quality. A crystalline solid is one in which the compound's molecules are arranged in a three-dimensional ordered lattice at the microscopic level. Polymorphism refers to the phenomenon whereby a single compound exists in multiple crystalline forms. A compound may exist in one or more crystalline forms, but the occurrence and properties of such forms cannot be specifically predicted. Changes in properties associated with different crystalline forms can be exploited to improve the final formulation. For example, such changes may increase solubility and thereby enhance bioavailability, improve the stability of the active ingredient, or, more unexpectedly, simultaneously increase solubility while providing good stability and reduced hygroscopicity. For the above-mentioned Compound I, it is necessary to further study and improve its different crystalline forms.

### Content of Invention

The technical problem addressed herein is to overcome the lack of pharmaceutically acceptable salts and crystalline forms of Compound I and to improve its physicochemical properties, thereby providing a pharmaceutically acceptable salt of the compound of formula (I), which may be a hydrate, an anhydrate, or a solvate crystal.

The present disclosure provides pharmaceutically acceptable salts of Compound I formed with pharmaceutically acceptable acids, the acids being selected from hydrochloric acid, hydrobromic acid, sulfuric acid, phosphoric acid, methanesulfonic acid, benzenesulfonic acid, p-toluenesulfonic acid, fumaric acid, maleic acid, oxalic acid, citric acid, tartaric acid, etc.

The present disclosure provides a pharmaceutically acceptable salt of Compound 1, wherein the molar ratio of pharmaceutically acceptable acid to Compound I is preferably 1:2-2:1, more preferably 1:1 or 2:1.

The pharmaceutically acceptable salt of Compound I provided herein is physicochemically stable under a range of storage conditions and is amenable to further processing.

The present disclosure provides pharmaceutically acceptable salts of Compound 1, preferably the p-toluenesulfonate, maleate, benzenesulfonate, phosphate, oxalate, diphenylsulfonate, or dihydrochloride.

The present disclosure provides a pharmaceutically acceptable salt of Compound I, preferably in crystalline solid form.

The present disclosure provides a pharmaceutically acceptable salt of Compound 1, preferably in anhydrous form.

The pharmaceutically acceptable salts of Compound I provided in the present disclosure include p-toluenesulfonate, maleate, benzenesulfonate, phosphate, oxalate, diphenylsulfonate, or dihydrochloride, preferably in crystalline solid form.

The p-toluenesulfonate and maleate provided in the present disclosure are preferably crystalline solids and, more preferably, anhydrates.

The present disclosure provides a maleate salt of Compound I.

Further, the present disclosure provides a crystalline form of the maleate salt of Compound I (hereinafter referred to as "maleate crystalline form A").

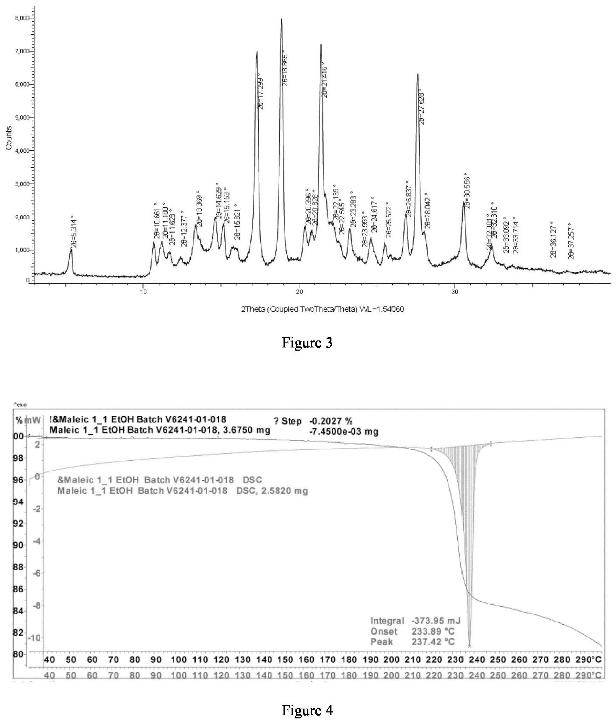

In some embodiments of the present disclosure, the X-ray powder diffraction (XRPD) pattern of maleate crystalline form A, using Cu-Kα radiation, is substantially as shown in Figure 3.

In some embodiments of the present disclosure, the thermogravimetric analysis (TGA) and differential scanning calorimetry (DSC) profiles of maleate crystalline form A are substantially as shown in Figure 4. The results indicate that maleate crystalline form A exhibits only 0.20% mass loss on heating to 120°C-essentially negligible- and an endothermic peak onset at approximately 233.9°C.

In some embodiments of the present disclosure, maleate crystalline form A is an anhydrate.

In some embodiments of the present disclosure, the X-ray powder diffraction pattern of maleate crystalline form A exhibits characteristic peaks at any one, two, three, four, five, six, seven, eight, nine, ten, eleven, twelve, thirteen, fourteen, fifteen, or sixteen of the following 20 values: 5.31°±0.2°, 10.66°±0.2°, 11.18°±0.2°, 13.37°±0.2°, 14.63°±0.2°, 15.15°±0.2°, 17.30°±0.2°, 18.87°±0.2°, 20.40°±0.2°, 21.42°±0.2°, 23.28°±0.2°, 24.62°±0.2°, 26.84°±0.2°, 27.63°±0.2°, 30.56°±0.2°, and 32.31°±0.2°.

In some embodiments of the present disclosure, the X-ray powder diffraction pattern of maleate crystalline form A exhibits a characteristic peak at 2θ = 5.31°±0.2° and two characteristic peaks at any two of the following 20 values: 17.30°±0.2°, 18.87°±0.2°, 21.42°±0.2°, 26.84°±0.2°, 27.63°±0.2°, and 30.56°±0.2°.

In some embodiments of the present disclosure, the X-ray powder diffraction pattern of maleate crystalline form A exhibits characteristic peaks at 20 = 5.31°±0.2° and 18.87±0.2°, and one additional characteristic peak at any one of 2θ = 17.30±0.2°, 21.42±0.2°, 26.84±0.2°, 27.63±0.2°, and 30.56±0.2°.

In some embodiments of the present disclosure, the X-ray powder diffraction pattern of maleate crystalline form A exhibits characteristic peaks at the following 20 values: 5.31°±0.2°, 17.30°±0.2°, 18.87°±0.2°, 21.42°±0.2°, and 27.63°±0.2°.

In some embodiments of the present disclosure, single crystal data for maleate crystalline form A are shown in Table 1.

**Table 1**

| | |
|---|---|
| C₄H₃O₄·C₂₁H₁₇F₃N₅O₂ | *Z* = 2 |
| *Mᵣ* = 543.46 | *F*(000) = 560 |
| Triclinic, *P*1 | *D*ₓ = 1.532 Mg m⁻³ |
| *a* = 8.7341 (6) Å | Cu *K*α radiation, λ = 1.54178 Å |
| *b* = 8.7807 (6) Å | Cell parameters from 9720 reflections |
| *c* = 16.8915 (12) Å | θ = 2.7-74.5° |
| α = 90.169 (4)° | µ = 1.09 mm⁻¹ |
| β = 101.366 (4)° | *T* = 170 K |
| γ = 111.411 (3)° | 0.11 × 0.04 × 0.02 mm |
| *V* = 1178.39 (14) Å³ | |

The present disclosure provides a hydrochloride salt of Compound I.

Further, the present disclosure provides a crystalline form of the dihydrochloride salt of Compound I (hereinafter referred to as "dihydrochloride crystalline form D").

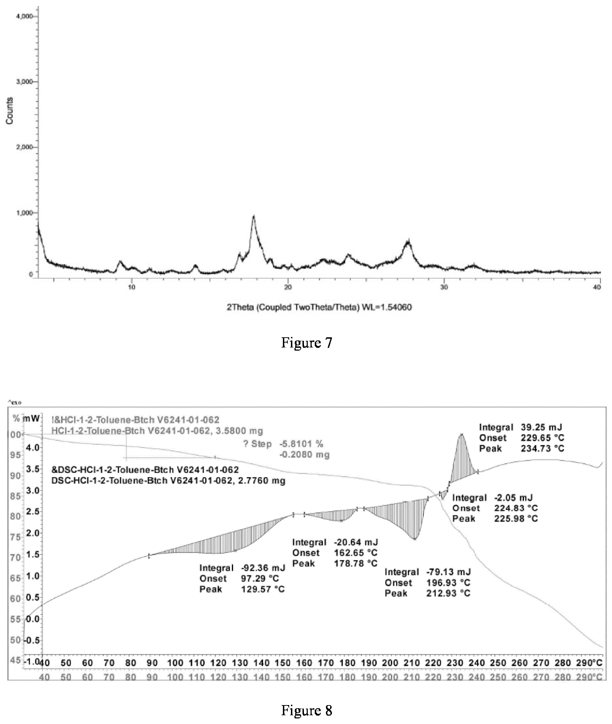

In some embodiments of the present disclosure, the X-ray powder diffraction pattern of dihydrochloride crystalline form D, using Cu-Kα radiation, is substantially as shown in Figure 7.

In some embodiments of the present disclosure, the thermogravimetric analysis and differential scanning calorimetry profiles of dihydrochloride crystalline form D are substantially as shown in Figure 8. The results indicate that the dihydrochloride crystalline form D begins to lose mass at approximately 30°C and shows multiple endothermic and exothermic signals.

In some embodiments of the present disclosure, the X-ray powder diffraction pattern of dihydrochloride crystalline form D exhibits characteristic peaks at any one, two, three, four, five, or six of the following 2θ values: 9.33°±0.2°, 14.05°±0.2°, 17.81°±0.2°, 18.86°±0.2°, 23.89°±0.2°, and 27.66°±0.2°.

The present disclosure provides a hydrobromide salt of Compound I.

Further, the present disclosure provides a crystalline form of the hydrobromide salt of Compound I (hereinafter referred to as "hydrobromide crystalline form A").

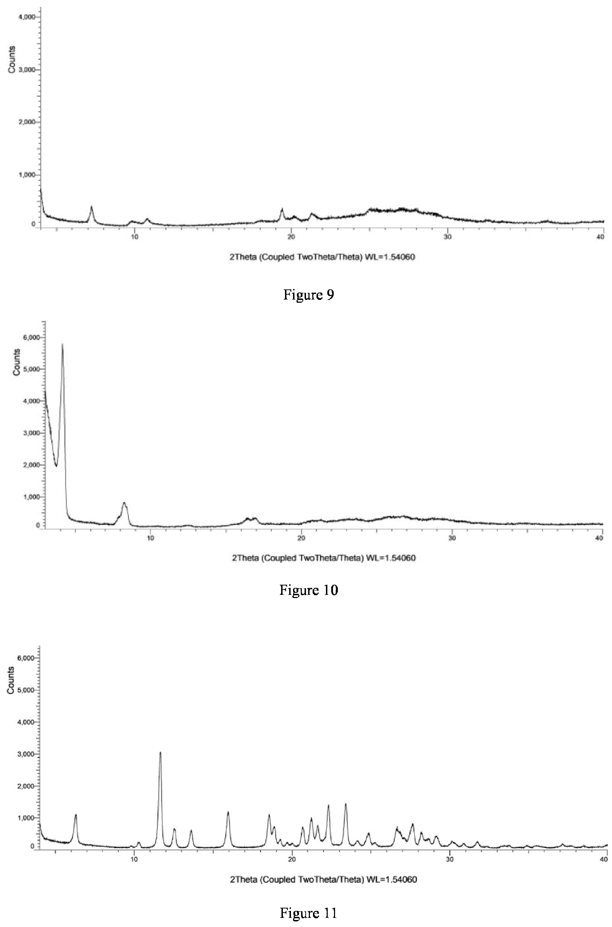

In some embodiments of the present disclosure, the X-ray powder diffraction pattern of hydrobromide crystalline form A, using Cu-Kα radiation, is substantially as shown in Figure 9.

In some embodiments of the present disclosure, the X-ray powder diffraction pattern of hydrobromide crystalline form A exhibits characteristic peaks at any one, two, three, four, five, six, seven, eight, or nine of the following 20 values: 3.54°±0.2°, 7.23°±0.2°, 9.88°±0.2°, 10.79°±0.2°, 19.42°±0.2°, 20.17°±0.2°, 21.34°±0.2°, 25.05°±0.2° and 32.58°±0.2°.

Further, the present disclosure provides a crystalline form of the dihydrobromide salt of Compound I (hereinafter referred to as "dihydrobromide crystalline form A").

In some embodiments of the present disclosure, the X-ray powder diffraction pattern of dihydrobromide crystalline form A, using Cu-Kα radiation, is substantially as shown in Figure 10.

In some embodiments of the present disclosure, the X-ray powder diffraction pattern of dihydrobromide crystalline form A exhibits characteristic peaks at any one, two, three, four, five, six, or seven of the following 20 values: 4.15°±0.2°, 7.84°±0.2°, 8.27°±0.2°, 12.43°±0.2°, 15.64°±0.2°, 16.44°±0.2°, and 16.89°±0.2°.

The present disclosure provides a phosphate salt of Compound I.

Further, the present disclosure provides a crystalline form of the phosphate salt of Compound I (hereinafter referred to as "phosphate crystalline form A").

In some embodiments of the present disclosure, the X-ray powder diffraction pattern of phosphate crystalline form A, using Cu-Kα radiation, is substantially as shown in Figure 11.

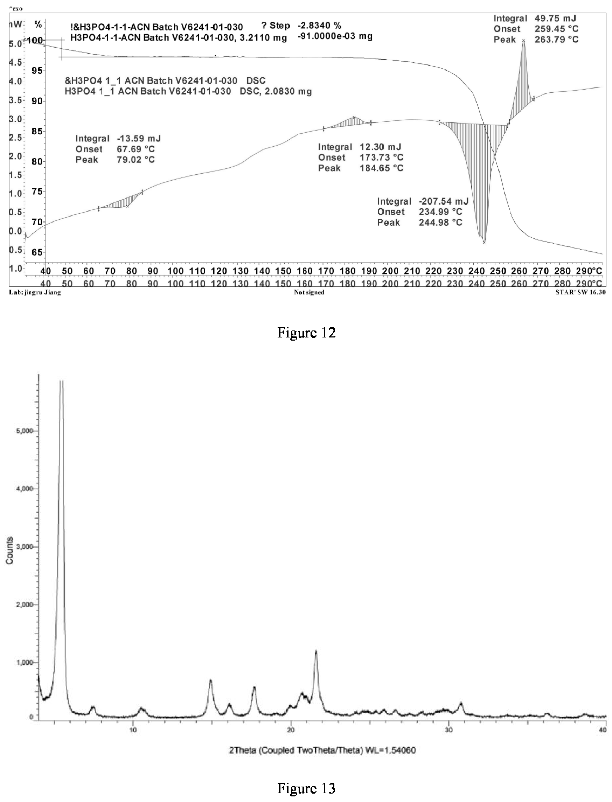

In some embodiments of the present disclosure, the thermogravimetric analysis and differential scanning calorimetry profiles of phosphate crystalline form A are substantially as shown in Figure 12. The results indicate that phosphate crystalline form A exhibits only 2.83% mass loss on heating to 120°C and the first endothermic peak onset at approximately 67.7°C.

In some embodiments of the present disclosure, the X-ray powder diffraction pattern of phosphate crystalline form A exhibits characteristic peaks at any one, two, three, four, five, six, seven, eight, nine, ten, eleven, twelve, thirteen, or fourteen of the following 20 values: 6.26°±0.2°, 11.66°±0.2°, 12.54°±0.2°, 13.62°±0.2°, 15.95°±0.2°, 18.54°±0.2°, 18.83°±0.2°, 20.69°±0.2°, 21.24°±0.2°, 21.65°±0.2°, 22.32°±0.2°, 23.42°±0.2°, 24.86°±0.2°, and 27.63°±0.2°.

The present disclosure provides a benzenesulfonate salt of Compound I.

Further, the present disclosure provides a crystalline form of the benzenesulfonate salt of Compound I (hereinafter referred to as "benzenesulfonate crystalline form A").

In some embodiments of the present disclosure, the X-ray powder diffraction pattern of benzenesulfonate crystalline form A, using Cu-Kα radiation, is substantially as shown in Figure 13.

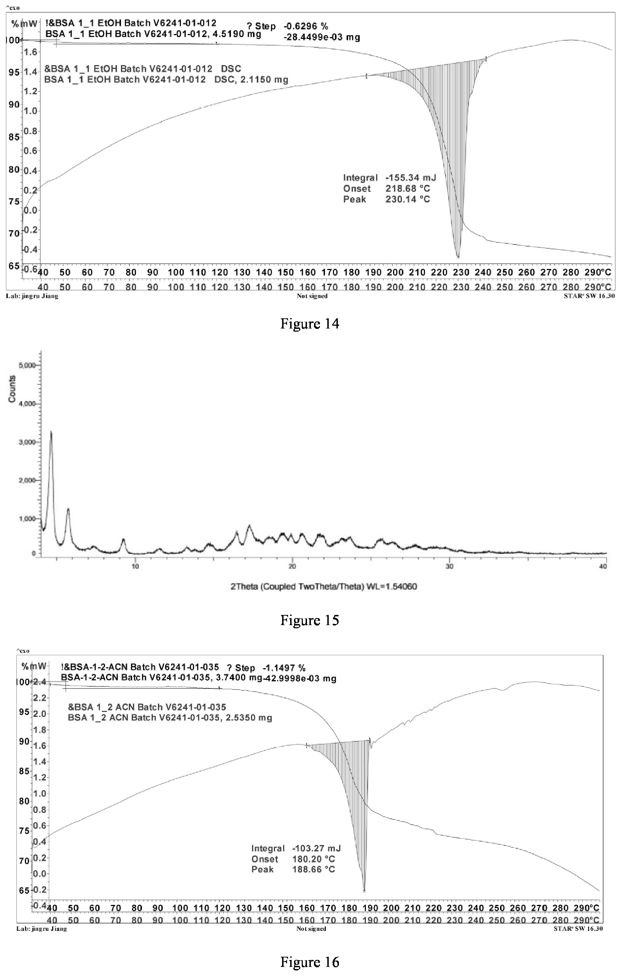

In some embodiments of the present disclosure, the thermogravimetric analysis and differential scanning calorimetry profiles of benzenesulfonate crystalline form A are substantially as shown in Figure 14. The results indicate that benzenesulfonate crystalline form A exhibits only about 0.63% mass loss on heating to 120°C and the first endothermic peak onset at approximately 218.7°C.

In some embodiments of the present disclosure, benzenesulfonate crystalline form A is an anhydrate.

In some embodiments of the present disclosure, the X-ray powder diffraction pattern of benzenesulfonate crystalline form A exhibits characteristic peaks at any one, two, three, four, five, six, seven, or eight of the following 20 values: 5.48°±0.2°, 7.46°±0.2°, 10.58°±0.2°, 14.93°±0.2°, 16.09°±0.2°, 17.66°±0.2°, 21.61°±0.2°, and 30.75°±0.2°.

The present disclosure provides a diphenylsulfonate salt of Compound I.

Further, the present disclosure provides a crystalline form of the diphenylsulfonate salt of Compound I (hereinafter referred to as "diphenylsulfonate crystalline form A").

In some embodiments of the present disclosure, the X-ray powder diffraction pattern of diphenylsulfonate crystalline form A, using Cu-Kα radiation, is substantially as shown in Figure 15.

In some embodiments of the present disclosure, the thermogravimetric analysis and differential scanning calorimetry profiles of diphenylsulfonate crystalline form A are substantially as shown in Figure 16. The results indicate that diphenylsulfonate crystalline form A exhibits only about 1.15% mass loss on heating to 120°C and an endothermic peak onset at approximately 180.2°C.

In some embodiments of the present disclosure, diphenylsulfonate crystalline form A is an anhydrate.

In some embodiments of the present disclosure, the X-ray powder diffraction pattern of diphenylsulfonate crystalline form A exhibits characteristic peaks at any one, two, three, four, five, six, seven, or eight of the following 20 values: 4.66°±0.2°, 5.73°±0.2°, 9.23°±0.2°, 16.44°±0.2°, 17.27°±0.2°, 19.93°±0.2°, 20.64°±0.2°, and 21.77°±0.2°.

The present disclosure provides a p-toluenesulfonate salt of Compound I.

Further, the present disclosure provides a crystalline form of the p-toluenesulfonate salt of Compound I (hereinafter referred to as "p-toluenesulfonate crystalline form A").

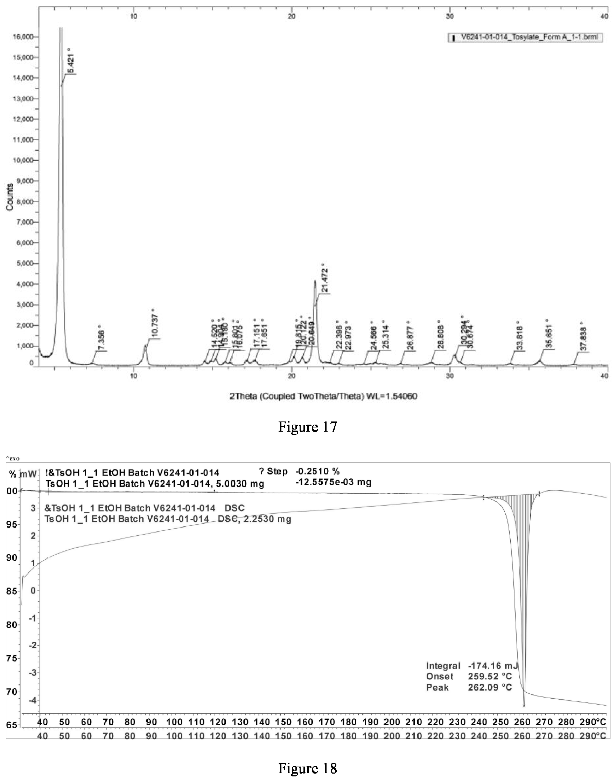

In some embodiments of the present disclosure, the X-ray powder diffraction pattern of p-toluenesulfonate crystalline form A, using Cu-Kα radiation, is substantially as shown in Figure 17.

In some embodiments of the present disclosure, the thermogravimetric analysis and differential scanning calorimetry profiles of p-toluenesulfonate crystalline form A are substantially as shown in Figure 18. The results indicate that p-toluenesulfonate crystalline form A exhibits almost no mass loss on heating to 120°C and an endothermic peak onset at approximately 259.5°C.

In some embodiments of the present disclosure, p-toluenesulfonate crystalline form A is an anhydrate.

In some embodiments of the present disclosure, the X-ray powder diffraction pattern of p-toluenesulfonate crystalline form A exhibits characteristic peaks at any one, two, three, four, five, six, seven, eight, nine, or ten of the following 20 values: 5.42°±0.2°, 10.74°±0.2°, 14.52°±0.2°, 15.18°±0.2°, 17.15°±0.2°, 17.65°±0.2°, 20.12°±0.2°, 20.65°±0.2°, 21.47°±0.2°, and 30.29°±0.2°.

In some embodiments of the present disclosure, the X-ray powder diffraction pattern of p-toluenesulfonate crystalline form A exhibits characteristic peaks at the following 20 values: 5.42°±0.2°, 10.74°±0.2°, and 21.47°±0.2°.

The present disclosure provides an oxalate salt of Compound I.

Further, the present disclosure provides a crystalline form of the oxalate salt of Compound I (hereinafter referred to as "oxalate crystalline form A").

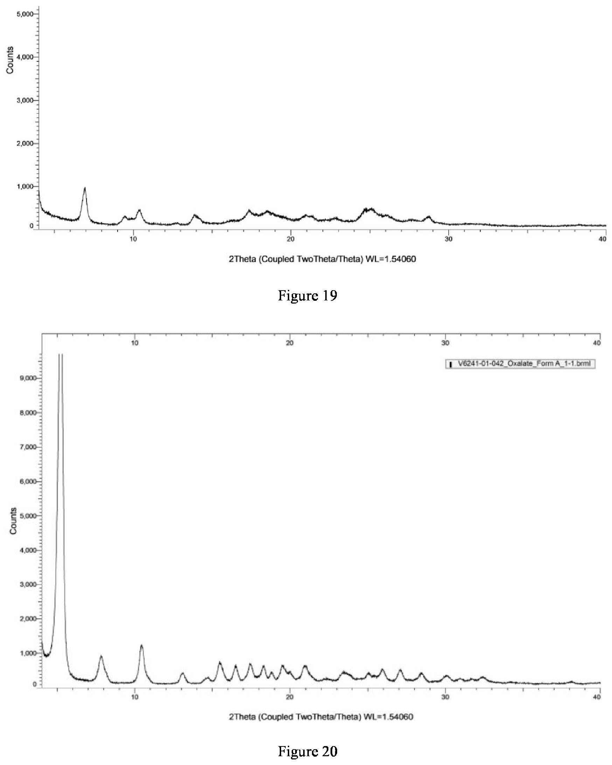

In some embodiments of the present disclosure, the X-ray powder diffraction pattern of oxalate crystalline form A, using Cu-Kα radiation, is substantially as shown in Figure 20.

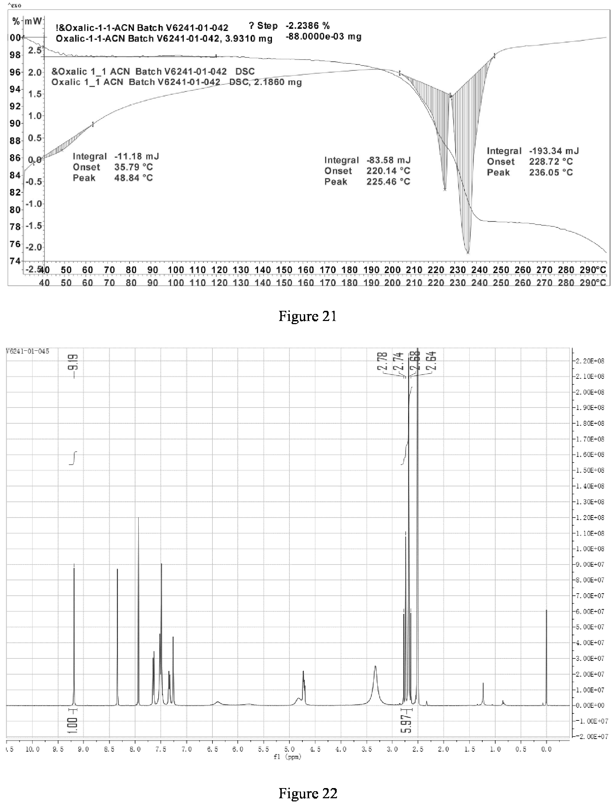

In some embodiments of the present disclosure, the thermogravimetric analysis and differential scanning calorimetry profiles of oxalate crystalline form A are substantially as shown in Figure 21. The results indicate that oxalate crystalline form A exhibits about 2.24% mass loss on heating to 120°C and the first endothermic peak appears at approximately 35.8°C.

In some embodiments of the present disclosure, the X-ray powder diffraction pattern of oxalate crystalline form A exhibits characteristic peaks at any one, two, three, four, five, six, seven, eight, nine, ten, eleven, twelve, or thirteen of the following 20 values: 5.20°±0.2°, 7.85°±0.2°, 10.46°±0.2°, 13.11°±0.2°, 15.52°±0.2°, 16.51°±0.2°, 17.46°±0.2°, 18.29°±0.2°, 19.56°±0.2°, 20.98°±0.2°, 25.95°±0.2°, 27.11°±0.2°, and 28.46°±0.2°.

The present disclosure provides a pharmaceutical composition comprising a therapeutically and/or prophylactically effective amount of a pharmaceutically acceptable salt of Compound I disclosed herein, together with pharmaceutically acceptable excipients.

The present disclosure provides the use of a pharmaceutically acceptable salt of Compound I in the manufacture of a pharmaceutical composition for treating diseases amenable to treatment with PRMT5 inhibitors.

### Technical Effect

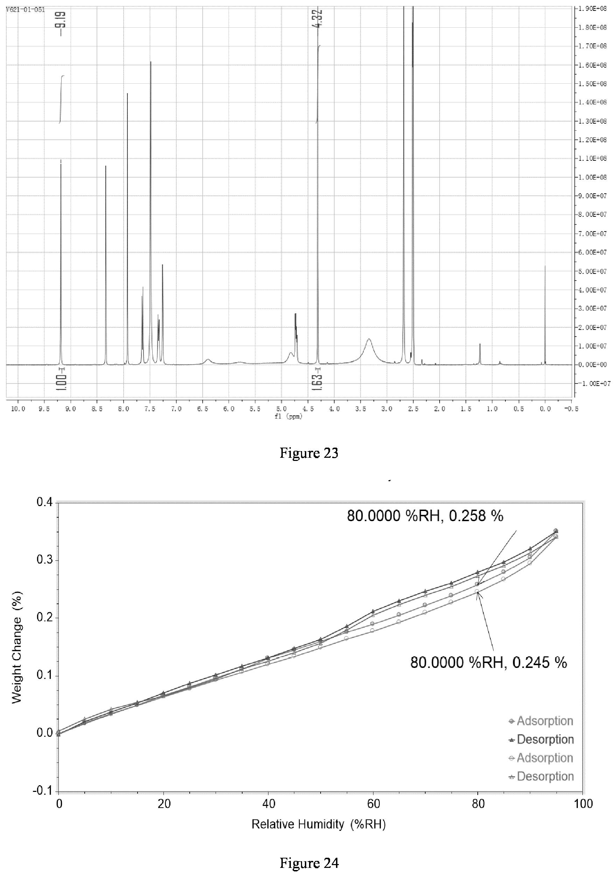

The present application investigated the formation of salts and crystalline forms of Compound I with sulfuric acid, methanesulfonic acid, fumaric acid, citric acid, and tartaric acid. The experimental results indicated that only mixtures of salts of Compound I and the respective acids were obtained, which are unsuitable for further development. For example, Compound I and sulfuric acid did not yield sulfate salt crystals in an ethanol system; reaction with methanesulfonic acid produced different crystalline forms at different feed molar ratios, making control of a specific form very challenging; at a 1:1 molar ratio of Compound I to fumaric acid, only a mixture of Compound I and the fumarate salt was obtained, and alternative methods yielded only fumarate with very low crystallinity (XRPD shown in Figure 19); Compound I with citric acid only formed a mixture of Compound I and the citrate salt (¹H-NMR results shown in Figure 22); and Compound I with tartaric acid likewise only formed a mixture of Compound I and the tartrate salt (¹H-NMR results shown in Figure 23).

To obtain salt forms and crystals of Compound I suitable for development, the inventors of the present application further investigated reactions of Compound I with acids such as maleic acid, p-toluenesulfonic acid, phosphoric acid, and oxalic acid. The studies found that the salts formed with these acid ligands and Compound I exhibit good stability.

### Description of Attached Drawings

Figure 1 Powder Appearance of Maleate Crystalline Form A
Figure 2 PLM Image of Maleate Crystalline Form A
Figure 3 XRPD Pattern of Maleate Crystalline Form A
Figure 4 TGA and DSC Profiles of Maleate Crystalline Form A
Figure 5 ¹H-NMR Spectrum of Maleate Crystalline Form A
Figure 6 Ellipsoid Plot of the Three-dimensional Molecular Structure of Maleate Crystalline Form A
Figure 7 XRPD Pattern of Dihydrochloride Crystalline Form D
Figure 8 TGA and DSC Profiles of Dihydrochloride Crystalline Form D
Figure 9 XRPD Pattern of Hydrobromide Crystalline Form A
Figure 10 XRPD Pattern of Dihydrobromide Crystalline Form A
Figure 11 XRPD Pattern of Phosphate Crystalline Form A
Figure 12 TGA and DSC Profiles of Phosphate Crystalline Form A
Figure 13 XRPD Pattern of Benzenesulfonate Crystalline Form A
Figure 14 TGA and DSC Profiles of Benzenesulfonate Crystalline Form A
Figure 15 XRPD Pattern of Diphenylsulfonate Crystalline Form A
Figure 16 TGA and DSC Profiles of Diphenylsulfonate Crystalline Form A
Figure 17 XRPD Pattern of p-Toluenesulfonate Crystalline Form A
Figure 18 TGA and DSC Profiles of p-Toluenesulfonate Crystalline Form A
Figure 19 XRPD Pattern of Fumarate Crystalline Form A
Figure 20 XRPD Pattern of Oxalate Crystalline Form A
Figure 21 TGA and DSC Profiles of Oxalate Crystalline Form A
Figure 22 ¹H-NMR Spectrum of Mixture of Citrate and Free Base
Figure 23 ¹H-NMR Spectrum of Mixture of Tartrate and Free Base
Figure 24 DVS Profile of Maleate Crystalline Form A
Figure 25 XRPD Comparison of Maleate Crystalline Form A after DVS Test (Top: after DVS Test, Bottom: before DVS Test)
Figure 26 Comparison of Stability of Maleate Crystalline Form A after Storage under Different Conditions (top to bottom: 25°C/60% RH for 4 weeks, 25°C/60% RH for 2 weeks, 60°C for 4 weeks, 60°C for 2 weeks, 40°C/75% RH for 4 weeks, 40°C/75% RH for 2 weeks, reference maleate crystalline form A)
Figure 27 XRPD Comparison of Maleate Crystalline Form A before and after Jet Milling (top: after milling; bottom: before milling)
Figure 28 PLM Image of Maleate Crystalline Form A after Jet Milling
Figure 29 Stability of Maleate Crystalline Form A in the Formulation (top to bottom: 9.2 kN pressure; 4.2 kN pressure;
maleate crystalline form A and microcrystalline cellulose mixed powder; microcrystalline cellulose; reference maleate crystalline form A)
Figure 30 XRPD Comparison of p-Toluenesulfonate Crystalline Form A after DVS Test (Top: after DVS Test, Bottom: before DVS Test)
Figure 31 Comparison of Stability of p-Toluenesulfonate Crystalline Form A after Storage under Different Conditions (top to bottom: 25°C/60% RH for 4 weeks, 25°C/60% RH for 2 weeks, 60°C for 4 weeks, 60°C for 2 weeks, 40°C/75% RH for 4 weeks, 40°C/75% RH for 2 weeks, reference p-toluenesulfonate crystalline form A)

### Specific Implementations

The present disclosure is further described in detail based on the following examples, which set forth the preparation and use methods of the crystalline forms disclosed herein. It is obvious to those skilled in the art that numerous modifications to the materials and methods described herein can be made without departing from the scope of the present disclosure.

The abbreviations used in the present disclosure are explained as follows:
XRPD: X-ray Powder Diffraction
DSC: Differential Scanning Calorimetry
TGA: Thermogravimetric Analysis
¹HNMR: Hydrogen-1 Nuclear Magnetic Resonance Spectroscopy
RH: Relative Humidity
PLM: Polarized Light Microscopy
DVS: Dynamic Vapor Sorption
HPLC: High Performance Liquid Chromatography

The XRPD pattern was obtained using a Bruker D8 Advance diffractometer. Before the experiment, the voltage was set to 40 KV and the current to 40 mA. The sample was loaded onto a zero-background sample holder and scanned over an angular range of 2°-40°. The scan step size was set to 0.01°, and the wavelength of the X-radiation used for measurement was 1.54060 Å.

Single crystal data for maleate were obtained using Bruker D8 Venture. The instrument parameters are shown in Table 2:

**Table 2**

| | |
|---|---|
| Light source: Cu target | X-ray: Cu-Kα (λ = 1.54178 Å) |
| Detector: CMOS area detector | Resolution: 0.80 Å |
| Voltage/current: 50 kV, 1.2 mA | Exposure time: 30 s |
| Detector-sample distance: 40 mm | Test temperature: 170(2) K |

After integrating and reducing the diffraction data with the SAINT program, empirical absorption corrections were applied using SADABS. The single-crystal structure was solved by direct methods using SHELXT2014 and refined by least-squares refinement. Hydrogen atoms were refined isotropically; hydrogen atoms on C-H were generated by calculation and refined using a riding model. The Flack parameter is 0.05(12), and C8 is assigned the S configuration.

The differential scanning calorimetry experiments were conducted using a Mettler-Toledo DSC3 differential scanning calorimeter. Before the experiment, the heating rate and melting enthalpy were calibrated using indium as a reference material. The sample was placed into a standard 40 µL aluminum crucible and covered with a perforated lid. The weight of the sample was recorded accurately. The sample tray containing the sample was placed in the sample chamber. A standard 40 µL aluminum crucible configured in the same manner but without a sample was placed in the reference position. DSC measurements were performed by heating the sample from 30°C to 300°C at a heating rate of 10°C/min. Nitrogen purge was performed during the experiment at a flow rate of 50 mL/min.

TGA data were collected using Mettler-Toledo TGA 2. Before sample analysis, TGA 2 was calibrated with nickel. Samples were placed in open aluminum pans, weighed automatically, and loaded into the TGA furnace. The furnace was heated from 31°C to 300°C at 10°C/min under a nitrogen flow of 20 mL/min.

Moisture adsorption and desorption data were obtained using a dynamic vapor sorption device (Model TA Discovery SA). Samples were equilibrated at 25°C/0% RH for 300 min at the start of the experiment. Thereafter, humidity was varied from 0% to 95% RH in 5% RH increments, with nitrogen used to control and maintain the humidity. Equilibration during testing was defined as a mass change rate of < 0.02% per minute over 10 min, with a maximum equilibration time of 120 min.

The data of the samples were collected using a Bruker 400 MHz NMR spectrometer with DMSO-d₆ as the solvent.

The preparation method of simulated gastrointestinal fluid involved in the present disclosure is:
SGF: Transfer 2.02 g of sodium chloride into a 1000 mL beaker and add 950 mL of purified water to dissolve. Next, adjust the pH to 1.2 with 12 N hydrochloric acid. Transfer the solution to a 1000 mL volumetric flask and make up to 1000 mL with purified water.

FeSSIF: Transfer 4.04 g of sodium hydroxide, 8.65 g of acetic acid, and 11.87 g of sodium chloride into a 1000 mL volumetric flask, and dissolve with 950 mL of purified water. Titrate the obtained solution to pH 5.0 with 1M sodium hydroxide or 1M hydrochloric acid aqueous solution, and then make up to 1000 mL with purified water. Finally, weigh 11.20 g of SIF powder, add it to the above 1000 mL of pH 5.0 solution, and stir magnetically until clear.

FaSSIF: Transfer 0.42 g of sodium hydroxide, 3.44 g of anhydrous sodium dihydrogen phosphate, and 6.19 g of sodium chloride into a 1000 mL volumetric flask, and add 950 mL of purified water to dissolve. Titrate the obtained solution to pH 6.5 with 1M sodium hydroxide or 1M hydrochloric acid aqueous solution, and then make up to 1000 mL with purified water. Finally, weigh 2.240 g of SIF powder, add it to the above 1000 mL of pH 6.5 solution, and stir magnetically until dissolved.

The process parameters of jet milling are shown in Table 3.

**Table 3**

| Pressure/Temperature | Parameter Setting |
|---|---|
| Feed Pressure | 5.5 bar |
| Temperature | 23 °C |
| Milling Pressure | 5.5 bar |

The solubility testing method described in the present application is shown in Table 4.

**Table 4**

| Column | Waters Xselect HSS T3 (4.6 mm*150 mm, 3.5 µm) | | | |
|---|---|---|---|---|
| Wavelength | 254 nm | | | |
| Column temperature | 40 °C | | | |
| Flow rate | 1.0 mL/min | | | |
| Injection volume | 5.0 µL | | | |
| Diluent/needle wash | 50% ACN/50%H₂O (1v/1v) | | | |
| Sample concentration | 0.0005-0.5 mg/mL | | | |
| Sample tray temperature | Room temperature | | | |

| Mobile phase | A:0.05% TFA in H₂O | | B: ACN | |
|---|---|---|---|---|
| Gradient elution | Time (min) | %A | | %B |
| | 0.00 | 80.0 | | 20.0 |
| | 10.00 | 10.0 | | 90.0 |
| | 10.01 | 80.0 | | 20.0 |
| | 12.00 | 80.0 | | 20.0 |

The high performance liquid chromatography (HPLC) method for determining enantiomeric excess (%) used in the present application is shown in Table 5.

**Table 5**

| Column | CHIRALPAK IC (250 mm*4.6 mm, 5 µm) | | | |
|---|---|---|---|---|
| Wavelength | 254 nm | | | |
| Column temperature | 25 °C | | | |
| Flow rate | 1.0 mL/min | | | |
| Injection volume | 2.0 µL | | | |
| Diluent/needle wash | MeOH | | | |

| Mobile phase | A: Hexane | | B: EtOH | |
|---|---|---|---|---|
| Gradient elution | Time (min) | A% | | B% |
| | 0.0 | 50 | | 50 |
| | 30 | 50 | | 50 |

The purity testing method described in the present application is shown as follows.

**Table 6**

| Column | Xbridge Shield RP18 (4.6mm*150mm, 3.5µm) | | | |
|---|---|---|---|---|
| Wavelength | 238 nm | | | |
| Column temperature | 40 °C | | | |
| Flow rate | 1.0 mL/min | | | |
| Injection volume | 5.0 µL | | | |
| Diluent/needle wash | 50% ACN/50%H₂O (1v/1v) | | | |
| Sample concentration | 0.5 mg/mL | | | |
| Sample tray temperature | Room temperature | | | |

| Mobile phase | A: 5 MmolNH₄HCO₃/H₂O | | | B: ACN |
|---|---|---|---|---|
| Gradient elution | Time (min) | %A | %B | |
| | 0.00 | 95.0 | 5.0 | |
| | 20.00 | 5.0 | 95.0 | |
| | 20.01 | 95.0 | 5.0 | |
| | 25.00 | 95.0 | 5.0 | |

The term "centrifugation" refers to conventional methods in the art, such as centrifugation or filtration. The operation termed "centrifugation" is performed by placing the sample to be separated in a centrifuge tube and centrifuging at 10,000 rpm until all solids have settled to the bottom of the tube.

The term "drying" refers to conventional methods in the art, such as vacuum drying, hot-air drying, or airdrying. Drying temperature may be room temperature or higher, preferably room temperature to about 60°C, or about 50°C, or about 40°C. Drying time may be 2-48 h. Drying is carried out in a fume hood, hot-air oven, or vacuum oven.

The term "stirring" refers to conventional methods in the art, such as magnetic stirring or mechanical stirring, performed at 50-1800 rpm; preferably 300-900 rpm for magnetic stirring and 100-300 rpm for mechanical stirring.

The "room temperature" mentioned does not denote a specific temperature and refers to the range 10-30°C.

The "anhydrate" mentioned refers to a solid material that contains no crystalline water or crystalline solvent.

The "characteristic peak" mentioned refers to a representative diffraction peak used to identify a crystalline form. When measured with Cu-Kα radiation, peak positions typically may vary by ±0.2°.

In the present disclosure, the terms "crystal" and "crystalline form" may be characterized by X-ray powder diffraction. XRPD diffraction data of a crystal serve as a fingerprint, and those skilled in the art use the data to identify different crystalline forms. They typically select several representative peaks in the XRPD pattern as characteristic peaks to characterize a crystal, considering peak position, intensity, and shape.

However, those skilled in the art will appreciate that X-ray powder diffraction patterns may vary with instrument conditions, sample preparation, and sample purity. The intensity of diffraction peaks in the X-ray powder diffraction pattern may also vary with experimental conditions. As a matter of fact, it is related to the preferred orientation of the crystals. Accordingly, the diffraction peak intensities presented herein are illustrative and not intended for absolute comparison. Therefore, in determining whether two samples represent the same crystalline form, matching of peak positions within the above-specified error range is primary.

Those skilled in the art will appreciate that the X-ray powder diffraction patterns of the crystalline form claimed herein need not be completely identical to the X-ray powder diffraction patterns shown in the examples; any crystalline form exhibiting the same or substantially similar characteristic peaks as those shown herein falls within the scope of this disclosure. For example, where peak positions are indicated in an X-ray powder diffraction pattern, such indication encompasses any X-ray powder diffraction pattern having corresponding peaks within ±0.2° (2θ). Those skilled in the art can compare the X-ray powder diffraction patterns provided herein with the X-ray powder diffraction pattern of an unknown crystalline form to determine whether the two represent the same or different crystalline forms.

In some embodiments, the pharmaceutically acceptable salt of Compound I disclosed herein and its crystalline form are pure and substantially free of other crystalline forms. As used herein, "substantially free" when referring to a new crystalline form means the form contains less than 20% (w/w) of other crystalline forms, in particular less than 10% (w/w), more particularly less than 5% (w/w), and even more particularly less than 1% (w/w).

As used herein, the term "approximately", when referring to a measurable value (e.g., mass, time, temperature), indicates that there is a certain range of fluctuation around the specified value. This range can be ±10%, ±5%, ±1%, ±0.5%, or ±0.1%.

### Detailed Description of the Invention

### Example 1 Preparation of Maleate Crystalline Form A

A total of 500 mg of Compound I was transferred into a dry, clean 40 mL glass vial; 10 mL of anhydrous ethanol was added to the vial, and the mixture was stirred at 800 rpm for 1 h at room temperature to afford a clear solution; 614 µL of a 2 mol/L maleic acid solution was then added slowly, and stirring was continued at room temperature for 24 h. Subsequently, the solid was recovered by centrifugation and vacuum-dried at 40°C for 3 days to give maleate crystalline form A.

### Example 2 Characterization of Maleate Crystalline Form A

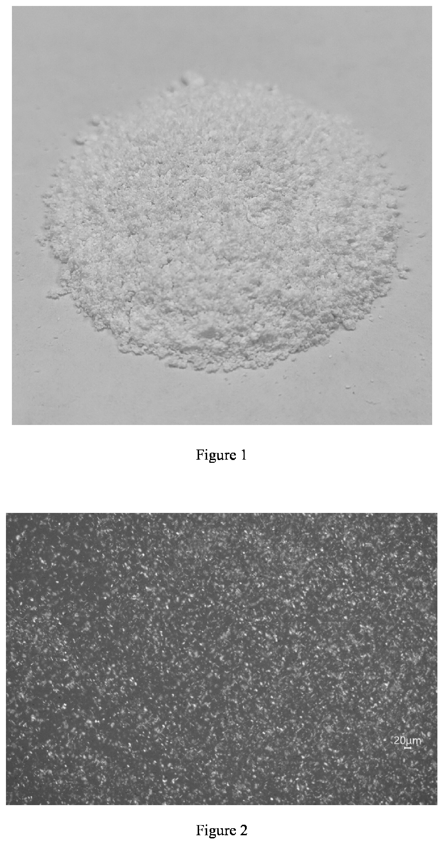

The appearance and PLM image of maleate crystalline form A are shown in Figure 1 and Figure 2, respectively.

The XRPD pattern of maleate crystalline form A is shown in Figure 3, and the XRPD data are shown in Table 7.

The TGA and DSC profiles of maleate crystalline form A are shown in Figure 4. The results showed that when heated from 31°C to 120°C, its mass loss was 0.20%. The DSC profile showed an endothermic peak onset at 233.89°C, which was a melting peak.

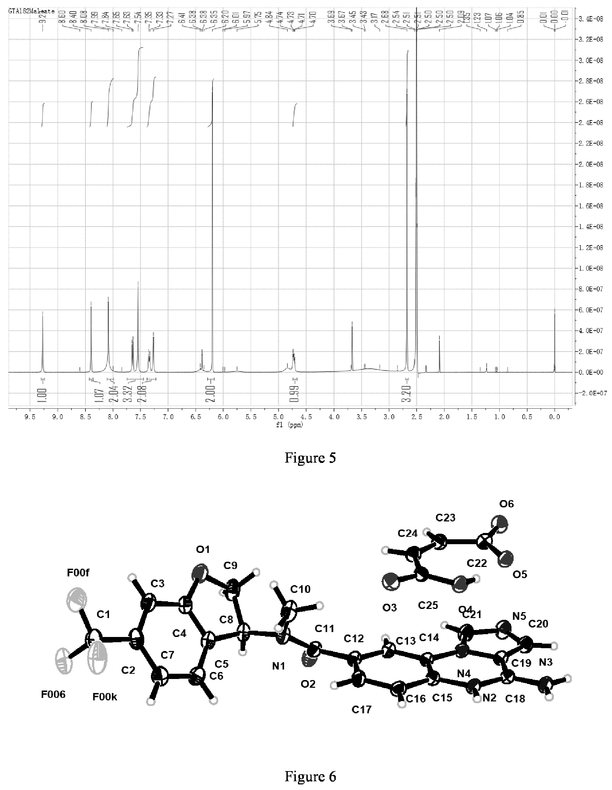

The ¹H-NMR spectrum of maleate crystalline form A is shown in Figure 5. The results showed a molar ratio of Compound I to maleic acid of 1:1 (mol/mol) and no obvious residual solvent.

**Table 7**

| Angle (°) | d Value (Å) | Rel. Intensity (%) |
|---|---|---|
| 5.31 | 16.62 | 9.2 |
| 10.66 | 8.29 | 13.3 |
| 11.18 | 7.91 | 12.1 |
| 11.63 | 7.60 | 6.7 |
| 12.38 | 7.15 | 3.9 |
| 13.37 | 6.62 | 14.6 |
| 14.63 | 6.05 | 18.1 |
| 15.15 | 5.84 | 15.0 |
| 15.82 | 5.60 | 5.0 |
| 17.30 | 5.12 | 85.8 |
| 18.87 | 4.70 | 100.0 |
| 20.40 | 4.35 | 13.8 |
| 20.83 | 4.26 | 12.9 |
| 21.42 | 4.15 | 91.1 |
| 22.14 | 4.01 | 16.3 |
| 22.55 | 3.94 | 7.8 |
| 23.28 | 3.82 | 13.0 |
| 23.99 | 3.71 | 1.6 |
| 24.62 | 3.61 | 11.3 |
| 25.52 | 3.49 | 8.3 |
| 26.84 | 3.32 | 21.0 |
| 27.63 | 3.23 | 77.8 |
| 28.04 | 3.18 | 14.3 |
| 30.56 | 2.92 | 27.6 |
| 32.00 | 2.79 | 3.4 |
| 32.31 | 2.77 | 10.6 |
| 33.09 | 2.70 | 1.6 |
| 33.71 | 2.66 | 1.7 |
| 36.13 | 2.48 | 1.2 |
| 37.26 | 2.41 | 0.8 |

### Example 3 Dihydrochloride Crystalline Form D

A total of 30 mg of Compound I was transferred into a 2 mL glass vial, 1 mL of toluene was added, and the mixture was stirred for 1 h. Subsequently, 71.95 µL of a 2N hydrochloric acid aqueous solution was slowly added to the reaction system, the mixture was stirred at room temperature for 24 h, and the solid was separated and vacuum-dried at 40°C for about 16 h to afford dihydrochloride crystalline form D.

The XRPD pattern of dihydrochloride crystalline form D, as shown in Figure 7, exhibited characteristic peaks at the following 20 values: 9.33°±0.2°, 14.05°±0.2°, 17.81°±0.2°, 18.86°±0.2°, 23.89°±0.2°, and 27.66°±0.2°.

The TGA and DSC profiles of dihydrochloride crystalline form D are shown in Figure 8. The results showed that dihydrochloride crystalline form D began to lose mass at approximately 30°C and exhibited multiple endothermic and exothermic signals.

### Example 4 Preparation of Hydrobromide Crystalline Form A

A total of 30 mg of Compound I was transferred into a 2 mL glass vial, 1 mL of ethanol was added, and the mixture was stirred for 1 h. Subsequently, 36.85 µL of a 2N hydrobromic acid aqueous solution was slowly added to the reaction system, the mixture was stirred at room temperature for 24 h, and the solid was separated and vacuum-dried at 40°C for about 16 h to afford hydrobromide crystalline form A.

The XRPD pattern of hydrobromide crystalline form A, as shown in Figure 9, exhibited characteristic peaks at the following 20 values: 3.54°±0.2°, 7.23°±0.2°, 9.88°±0.2°, 10.79°±0.2°, 19.42°±0.2°, 20.17°±0.2°, 21.34°±0.2°, 25.05°±0.2°, and 32.58°±0.2°.

### Example 5 Preparation of Dihydrobromide Crystalline Form A

A total of 30 mg of Compound I was transferred into a 2 mL glass vial, 1 mL of acetonitrile was added, and the mixture was stirred for 1 h. Subsequently, 71.95 µL of a 2N hydrobromic acid aqueous solution was slowly added to the reaction system, the mixture was stirred at room temperature for 24 h, and the solid was separated and vacuum-dried at 40°C for about 16 h to afford dihydrobromide crystalline form A.

The XRPD pattern of dihydrobromide crystalline form A, as shown in Figure 10, exhibited characteristic peaks at the following 20 values: 4.15°±0.2°, 7.84°±0.2°, 8.27°±0.2°, 12.43°±0.2°, 15.64°±0.2°, 16.44°±0.2°, and 16.89°±0.2°.

### Example 6 Preparation of Phosphate Crystalline Form A

A total of 30 mg of Compound I was transferred into a 2 mL glass vial, 1 mL of acetonitrile was added, and the mixture was stirred for 1 h. Subsequently, 36.85 µL of a 2N phosphoric acid aqueous solution was slowly added to the reaction system, the mixture was stirred at room temperature for 24 h, and the solid was separated and vacuum-dried at 40°C for about 16 h to afford phosphate crystalline form A.

The XRPD pattern of phosphate crystalline form A is shown in Figure 11, and the XRPD data are shown in Table 8.

The TGA and DSC profiles of phosphate crystalline form A are shown in Figure 12. The results showed that phosphate crystalline form A exhibited a 2.83% mass loss on heating from 31°C to 120°C and the first endothermic peak onset at approximately 67.7°C.

**Table 8**

| Angle (°) | d Value (Å) | Rel. Intensity (%) |
|---|---|---|
| 6.26 | 14.10 | 30.7 |
| 9.85 | 8.98 | 2.1 |
| 10.29 | 8.59 | 4.2 |
| 11.66 | 7.58 | 100.0 |
| 12.54 | 7.05 | 20.1 |
| 13.62 | 6.49 | 19.0 |
| 15.95 | 5.55 | 36.9 |
| 18.54 | 4.78 | 34.1 |
| 18.83 | 4.71 | 20.2 |
| 19.22 | 4.61 | 6.0 |
| 19.71 | 4.50 | 5.2 |
| 19.99 | 4.44 | 3.7 |
| 20.69 | 4.29 | 18.1 |
| 21.24 | 4.18 | 29.4 |
| 21.65 | 4.10 | 22.4 |
| 22.32 | 3.98 | 43.3 |
| 23.42 | 3.79 | 45.4 |
| 24.15 | 3.68 | 5.2 |
| 24.86 | 3.58 | 14.3 |
| 25.23 | 3.53 | 4.2 |
| 26.70 | 3.34 | 16.2 |
| 27.63 | 3.23 | 22.5 |
| 28.23 | 3.16 | 12.3 |
| 28.59 | 3.12 | 7.5 |
| 29.16 | 3.06 | 9.9 |
| 30.16 | 2.96 | 4.6 |
| 30.88 | 2.89 | 3.9 |
| 31.75 | 2.82 | 6.7 |
| 32.28 | 2.77 | 2.1 |
| 33.44 | 2.68 | 3.6 |
| 33.71 | 2.66 | 2.8 |
| 34.93 | 2.57 | 2.7 |
| 35.51 | 2.53 | 1.3 |
| 37.16 | 2.42 | 4.2 |
| 37.67 | 2.39 | 1.7 |
| 38.51 | 2.34 | 2.7 |

### Example 7 Preparation of Benzenesulfonate Crystalline Form A

A total of 30 mg of Compound I was transferred into a 2 mL glass vial, 1 mL of ethanol was added, and the mixture was stirred for 1 h. Subsequently, 36.85 µL of a 2N benzenesulfonic acid solution in methanol was slowly added to the reaction system, the mixture was stirred at room temperature for 24 h, and the solid was separated and vacuum-dried at 40°C for about 16 h to afford benzenesulfonate crystalline form A.

The XRPD pattern of benzenesulfonate crystalline form A is shown in Figure 13, and the XRPD data are shown in Table 9.

The TGA and DSC profiles of benzene sulfonate crystalline form A are shown in Figure 14. The results showed that benzenesulfonate crystalline form A exhibited a 0.6296% mass loss on heating from 31°C to 120°C, and the DSC profile showed an endothermic peak with an onset at approximately 218.7°C.

**Table 9**

| Angle (°) | d Value (Å) | Rel. Intensity (%) |
|---|---|---|
| 5.48 | 16.12 | 100.0 |
| 7.46 | 11.84 | 2.2 |
| 10.58 | 8.36 | 2.1 |
| 14.93 | 5.93 | 7.8 |
| 16.09 | 5.51 | 2.8 |
| 17.66 | 5.02 | 6.5 |
| 19.05 | 4.65 | 0.6 |
| 20.05 | 4.43 | 2.1 |
| 20.73 | 4.28 | 5.1 |
| 21.61 | 4.11 | 14.7 |
| 25.37 | 3.51 | 1.1 |
| 25.91 | 3.44 | 1.3 |
| 26.62 | 3.35 | 1.3 |
| 28.27 | 3.15 | 1.0 |
| 29.75 | 3.00 | 0.9 |
| 30.75 | 2.91 | 2.5 |
| 36.25 | 2.48 | 0.9 |
| 38.62 | 2.33 | 1.0 |

### Example 8 Preparation of Diphenylsulfonate Crystalline Form A

A total of 30 mg of Compound I was transferred into a 2 mL glass vial, 1 mL of acetonitrile was added, and the mixture was stirred for 1 h. Subsequently, 71.95 µL of a 2N benzenesulfonic acid solution in methanol was slowly added to the reaction system, the mixture was stirred at room temperature for 24 h, and the solid was separated and vacuum-dried at 40°C for about 16 h to afford diphenylsulfonate crystalline form A.

The XRPD pattern of diphenylsulfonate crystalline form A is shown in Figure 15, and the XRPD data are shown in Table 10.

The TGA and DSC profiles of diphenylsulfonate crystalline form A are shown in Figure 16. The results showed that the form exhibited a 1.1497% mass loss on heating from 31°C to 120°C, and the DSC profile showed an endothermic peak with an onset at approximately 180.20°C.

**Table 10**

| Angle (°) | d Value(Å) | Rel. Intensity (%) |
|---|---|---|
| 4.66 | 18.96 | 100.0 |
| 5.73 | 15.42 | 36.1 |
| 7.36 | 12.00 | 4.4 |
| 9.23 | 9.57 | 14.3 |
| 11.54 | 7.66 | 5.3 |
| 13.31 | 6.65 | 4.6 |
| 13.80 | 6.41 | 3.9 |
| 14.71 | 6.02 | 6.6 |
| 15.98 | 5.54 | 3.0 |
| 16.44 | 5.39 | 16.9 |
| 17.27 | 5.13 | 20.6 |
| 18.60 | 4.77 | 8.1 |
| 19.39 | 4.57 | 12.0 |
| 19.93 | 4.45 | 11.5 |
| 20.64 | 4.30 | 11.1 |
| 21.77 | 4.08 | 12.9 |
| 22.76 | 3.90 | 5.6 |
| 23.19 | 3.83 | 7.2 |
| 23.62 | 3.76 | 8.2 |
| 25.62 | 3.47 | 10.0 |
| 26.33 | 3.38 | 7.4 |
| 27.81 | 3.20 | 4.4 |
| 30.71 | 2.91 | 1.8 |

### Example 9 Preparation of p-Toluenesulfonate Crystalline Form A

A total of 30 mg of Compound I was transferred into a 2 mL glass vial, 1 mL of ethanol was added, and the mixture was stirred for 1 h. Subsequently, 36.85 µL of a 2N p-toluenesulfonic acid solution in methanol was slowly added to the reaction system, the mixture was stirred at room temperature for 24 h, and the solid was separated and vacuum-dried at 40°C for about 16 h to afford p-toluenesulfonate crystalline form A.

The XRPD pattern of p-toluenesulfonate crystalline form A is shown in Figure 17, and the XRPD data are shown in Table 11.

The TGA and DSC profiles of p-toluenesulfonate crystalline form A are shown in Figure 18. The results showed that the form exhibited almost no mass loss on heating to 120°C and an endothermic peak onset at 259.5°C.

By way of a non-limiting example, p-toluenesulfonate crystalline form A was an anhydrate.

**Table 11**

| Angle (°) | d Value (Å) | Rel. Intensity (%) |
|---|---|---|
| 5.42 | 16.29 | 100.0 |
| 7.36 | 12.01 | 0.4 |
| 10.74 | 8.23 | 5.1 |
| 14.52 | 6.10 | 1.0 |
| 14.90 | 5.94 | 1.1 |
| 15.18 | 5.83 | 1.6 |
| 15.80 | 5.60 | 0.9 |
| 16.08 | 5.51 | 0.8 |
| 17.15 | 5.17 | 1.3 |
| 17.65 | 5.02 | 1.2 |
| 19.82 | 4.48 | 0.9 |
| 20.12 | 4.41 | 2.2 |
| 20.65 | 4.30 | 1.8 |
| 21.47 | 4.14 | 21.1 |
| 22.40 | 3.97 | 0.5 |
| 22.97 | 3.87 | 0.4 |
| 24.57 | 3.62 | 0.4 |
| 25.31 | 3.52 | 0.7 |
| 26.88 | 3.31 | 0.5 |
| 28.81 | 3.10 | 0.8 |
| 30.29 | 2.95 | 2.4 |
| 30.67 | 2.91 | 0.8 |
| 33.82 | 2.65 | 0.5 |
| 35.65 | 2.52 | 1.3 |
| 37.84 | 2.38 | 0.3 |

### Example 10 Preparation of Oxalate Crystalline Form A

A total of 30 mg of Compound I was transferred into a 2 mL glass vial, 1 mL of acetonitrile was added, and the mixture was stirred for 1 h. Subsequently, 36.85 µL of a 2N oxalic acid solution in methanol was slowly added to the reaction system, the mixture was stirred at room temperature for 24 h, and the solid was separated and vacuum-dried at 40°C for about 16 h to afford oxalate crystalline form A.

The XRPD pattern of oxalate crystalline form A is shown in Figure 20, and the XRPD data are shown in Table 12.

The TGA and DSC profiles of oxalate crystalline form A are shown in Figure 21. The results showed that oxalate crystalline form A had a 2.2386% mass loss on heating from 31°C to 120°C. The DSC profile showed 3 endothermic events with an onset at approximately 35.8°C, 220.14°C, and 228.7°C, respectively.

**Table 12**

| Angle (°) | d Value (Å) | Rel. Intensity (%) |
|---|---|---|
| 5.20 | 16.98 | 100.0 |
| 7.85 | 11.26 | 6.8 |
| 10.46 | 8.45 | 9.6 |
| 13.11 | 6.75 | 2.7 |
| 14.67 | 6.03 | 1.2 |
| 15.52 | 5.71 | 4.3 |
| 16.51 | 5.37 | 4.2 |
| 17.46 | 5.08 | 3.7 |
| 18.29 | 4.85 | 3.2 |
| 18.82 | 4.71 | 1.9 |
| 19.56 | 4.53 | 3.3 |
| 20.05 | 4.43 | 1.9 |
| 20.98 | 4.23 | 3.8 |
| 22.33 | 3.98 | 1.2 |
| 23.52 | 3.78 | 1.9 |
| 25.08 | 3.55 | 1.8 |
| 25.95 | 3.43 | 2.8 |
| 27.11 | 3.29 | 2.7 |
| 28.46 | 3.13 | 2.6 |
| 30.08 | 2.97 | 1.8 |
| 30.96 | 2.89 | 1.0 |
| 31.73 | 2.82 | 0.7 |
| 32.40 | 2.76 | 1.3 |
| 38.10 | 2.36 | 0.7 |

### Example 11 Hygroscopicity of Maleate Crystalline Form A

High hygroscopicity can easily cause chemical degradation and crystalline form transformations of the active pharmaceutical ingredient (API), thereby directly affecting the API's physicochemical stability. In addition, strong hygroscopicity reduces the API's flowability, thus affecting its processing. On the other hand, highly hygroscopic drugs require maintenance of low humidity during manufacture and storage, imposing higher production requirements and incurring substantial costs.

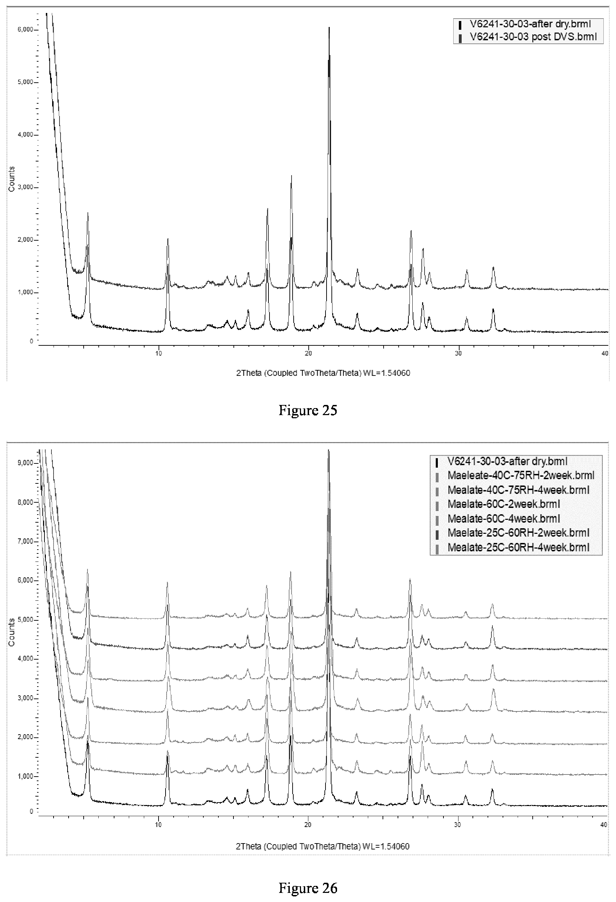

Maleate crystalline form A was subject to the DVS test, with results shown in Figure 24. From 0% RH to 80% RH, the hygroscopic weight gain was approximately 0.25%, indicating very low hygroscopicity. The samples after the DVS test were characterized by XRPD, as shown in Figure 25. The results showed that the crystalline form did not change after the DVS test, indicating that maleate crystalline form A has good humidity stability.

### Example 12 Physicochemical Stability of Maleate Crystalline Form A

### I. Experimental Method

For chemical stability studies, two open samples were prepared at each time point for each condition. Approximately 2 mg of maleate crystalline form A was transferred into a 20 mL glass vial, which was then covered with perforated aluminum foil to serve as an open sample. Samples were stored at 25°C/60% RH, 40°C/75% RH, and 60°C.

For physical stability studies, two open samples were prepared at each time point for each condition. Approximately 10 mg of maleate crystalline form A was transferred into a 20 mL glass vial, which was covered with perforated aluminum foil to serve as an open sample. Samples were stored at 25°C/60% RH, 40°C/75% RH, and 60°C.

All samples were stored for 4 weeks under the specified storage conditions. At predetermined time points (initial, 2 weeks, and 4 weeks), the corresponding samples were withdrawn and subject to chemical and physical evaluations, respectively. The physicochemical stability was assessed by XRPD and HPLC.

### II. Experimental Results

The physicochemical stability results of maleate crystalline form A stored at 25°C/60% RH, 40°C/75% RH, and 60°C are shown in Table 13, and the comparison of XRPD patterns before and after the test is shown in Figure 26.

**Table 13**

| Initial Crystalline Form | Storage Conditions | Storage Time | Appearance | Purity (%) | ee (%) | Crystalli ne Form Change |
|---|---|---|---|---|---|---|
| | - | Initial | Off-white powder | 99.66 | 100.00 | |
| | 40°C/75%RH (Open) | 2 weeks | Off-white powder | 99.69 | 100.00 | |
| | | 4 weeks | Off-white powder | 99.67 | 100.00 | |
| Maleate crystalline form A | 60 °C (Open) | 2 weeks | Off-white powder | 99.69 | 100.00 | None |
| | | 4 weeks | Off-white powder | 99.67 | 100.00 | |
| | 25°C/60%RH (Open) | 2 weeks | Off-white powder | 99.68 | 100.00 | |
| | | 4 weeks | Off-white powder | 99.67 | 100.00 | |

High-temperature and high-humidity conditions arising from seasonal variation, regional climate differences, and environmental factors can affect the storage, transport, and manufacture of the API. Maleate crystalline form A exhibited good physicochemical stability after storage for 4 weeks at 25°C/60% RH, 40°C/75% RH, and 60°C, helping to avoid impacts on drug quality during storage due to crystal transformation or purity loss.

### Example 13 Mechanical Stability of Maleate Crystalline Form A

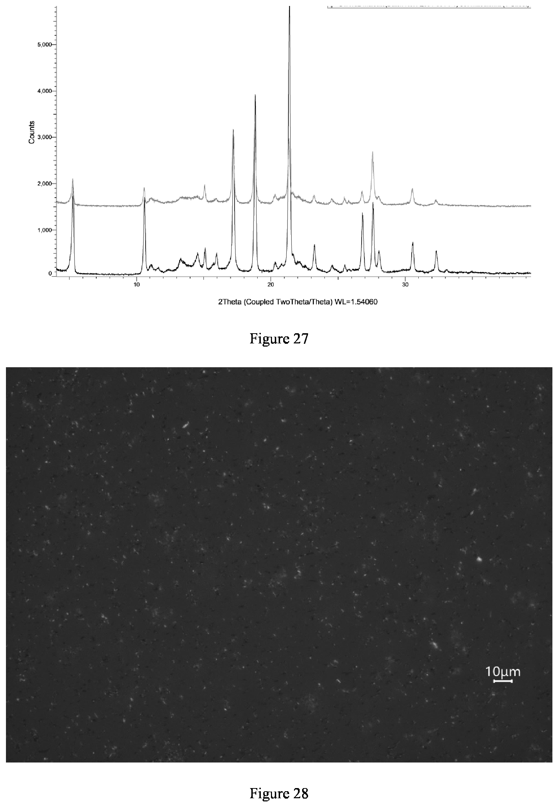

Jet milling: Powder of maleate crystalline form A was jet-milled using the process parameters listed in Table 3. The milled powder was then analyzed by XRPD and PLM, with results shown in Figure 27 and Figure 28, respectively. The results showed that, after jet milling, maleate crystalline form A retained its crystalline form and exhibited a significant reduction in crystal particle size.

Pressurization method: Maleate crystalline form A was weighed and transferred into a 5 mm-diameter die. The upper piston was then manually driven down into the die until a pressure of 40 MPa was reached and held for 1 min. Subsequently, the sample was removed and manually ground into a powder. Finally, the resulting powder was analyzed by XRPD. The results showed that maleate crystalline form A retained its crystalline form after pressurization.

### Example 14 Stability of Maleate Crystalline Form A in the Formulation

Equal amounts of maleate crystalline form A and microcrystalline cellulose PH102 were weighed to prepare a powder formulation comprising 50% (w/w) maleate crystalline form A, and the two components were mixed thoroughly.

A total of 200 mg of the uniformly mixed powder was transferred into an 8 mm-diameter die of a rotary tablet press. One tablet was compressed at 4.2 kN and another at 9.2 kN.

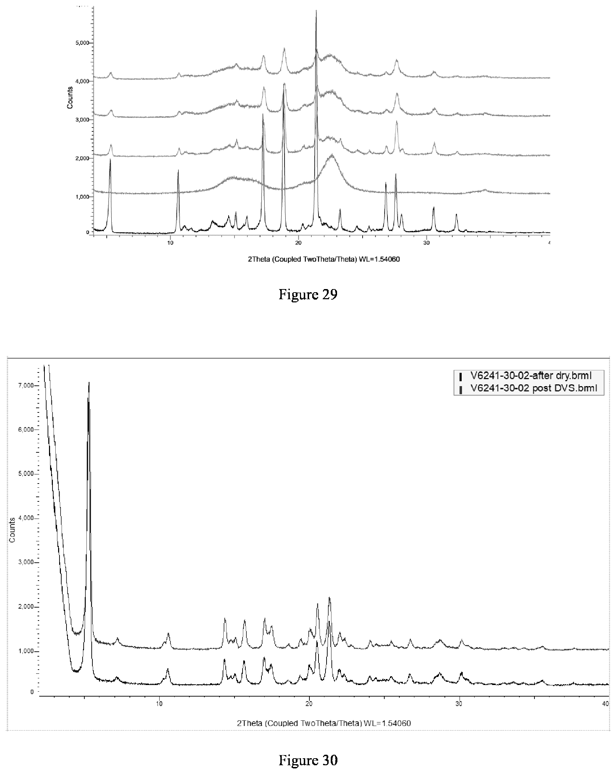

The resulting tablets were characterized for weight and hardness. The two tablets were then manually ground into powder respectively and analyzed by XRPD; the results are shown in Figure 29.

The results showed that even under relatively high pressures, the crystallinity of maleate crystalline form A in the formulation remained relatively stable.

### Example 15 Solubility of Maleate Crystalline Form A

### I. Experimental Method

A total of 10 mg of maleate crystalline form A was transferred into a dry, clean 4 mL glass vial. Then, 1 mL of simulated gastric fluid (SGF), fed state simulated intestinal fluid (FeSSIF), or purified water was added to the vial. All vials were placed on a shaker and agitated at 200 rpm at 37°C.

At the predetermined time point (24 h), 200 µL of the sample was withdrawn from the vial into a centrifuge tube and centrifuged at 90,000 rpm for 5 min. An appropriate amount of the supernatant was then diluted and analyzed by HPLC to determine the solubility.

The remaining 800 µL of suspension in the vial was centrifuged at 8000 rpm for 10 min. The pH of the resulting supernatant was measured, and the precipitated solids were vacuum-dried at 40°C for approximately 16 h. The dried precipitated solids were then characterized by X-ray powder diffraction.

### II. Experimental Results

The dynamic solubility of maleate crystalline form A in different media is shown in Table 14.

**Table 14**

| Crystalline Form | Medium | 24 h | | |
|---|---|---|---|---|
| | | Solubility (µg/mL) | pH | Crystalline Form Change |
| | SGF | 442.88 | 0.96 | None |
| Maleate crystalline form A | FeSSIF | 257.62 | 4.83 | None |
| | H₂O | 42.12 | 4.17 | None |

The results showed that maleate crystalline form A exhibited high solubility in both SGF and FeSSIF, and that after 24 h solubility testing in SGF, FeSSIF, and water, its crystalline form remained unchanged, demonstrating good physical stability in these media.

Maleate crystalline form A exhibited high solubility, which is conducive to enhancing *in vivo* drug absorption and improving bioavailability; in addition, increased solubility may allow dose reduction while maintaining therapeutic efficacy, thereby reducing side effects and improving the safety of drugs.

### Example 16 Hygroscopicity of p-Toluenesulfonate Crystalline Form A

High hygroscopicity can easily cause chemical degradation and crystalline form transformations of the active pharmaceutical ingredient (API), thereby directly affecting the API's physicochemical stability. In addition, strong hygroscopicity reduces the API's flowability, thus affecting its processing. On the other hand, highly hygroscopic drugs require maintenance of low humidity during manufacture and storage, imposing higher production requirements and incurring substantial costs.

The DVS test of p-toluenesulfonate crystalline form A from 0% to 80% RH showed a hygroscopic weight gain of approximately 0.37%, indicating low hygroscopicity. The samples after the DVS test were characterized by XRPD, as shown in Figure 30. The results showed that the crystalline form did not change after the DVS test, indicating that p-toluenesulfonate crystalline form A has good humidity stability.

### Example 17 Solubility of p-Toluenesulfonate Crystalline Form A

### I. Experimental Method

A total of 10 mg of p-toluenesulfonate crystalline form A was transferred into a dry, clean 4 mL glass vial. Then, 1 mL of simulated gastric fluid (SGF), fed state simulated intestinal fluid (FeSSIF), or purified water was added to the vial. All vials were placed on a shaker and agitated at 200 rpm at 37°C.

At the predetermined time point (24 h), 200 µL of the sample was withdrawn from the vial into a centrifuge tube and centrifuged at 90,000 rpm for 5 min. An appropriate amount of the supernatant was then diluted and analyzed by high performance liquid chromatography to determine the solubility.

The remaining 800 µL of suspension in the vial was centrifuged at 8000 rpm for 10 min. The pH of the resulting supernatant was measured, and the precipitated solids were vacuum-dried at 40°C for approximately 16 h. The dried precipitated solids were then characterized by X-ray powder diffraction.

### II. Experimental Results

The dynamic solubility of p-toluenesulfonate crystalline form A in different media is shown in Table 15.

**Table 15**

| Crystalline Form | Medium | 24 h | | |
|---|---|---|---|---|
| | | Solubility (µg/mL) | pH | Crystalline Form Change |
| p-toluenesulfonate crystalline form A | SGF | 293.98 | 0.98 | None |
| | FeSSIF | 365.38 | 4.78 | None |
| | H₂O | 248.78 | 3.19 | None |

The results showed that p-toluenesulfonate crystalline form A exhibited high solubility in SGF, FeSSIF, and water, and after 24 h solubility testing in SGF, FeSSIF, and water, its crystalline form remained unchanged, demonstrating good physical stability in these media.

p-Toluenesulfonate crystalline form A exhibited high solubility, which is conducive to enhancing *in vivo* drug absorption and improving bioavailability; in addition, increased solubility may allow dose reduction while maintaining therapeutic efficacy, thereby reducing side effects and improving the safety of drugs.

### Example 18 Physicochemical Stability of p-Toluenesulfonate Crystalline Form A

### I. Experimental Method

For chemical stability studies, two open samples were prepared at each time point for each condition. Approximately 2 mg of p-toluenesulfonate crystalline form A was transferred into a 20 mL glass vial, which was then covered with perforated aluminum foil to serve as an open sample. Samples were stored at 25°C/60% RH, 40°C/75% RH, and 60°C.

For physical stability studies, two open samples were prepared at each time point for each condition. Approximately 10 mg of p-toluenesulfonate crystalline form A was transferred into a 20 mL glass vial, which was covered with perforated aluminum foil to serve as an open sample. Samples were stored at 25°C/60% RH, 40°C/75% RH, and 60°C, respectively.

All samples were stored for 4 weeks under the specified storage conditions. At predetermined time points (initial, 2 weeks, and 4 weeks), the corresponding samples were withdrawn and subject to chemical and physical evaluations, respectively. The physicochemical stability was assessed by XRPD and HPLC.

### II. Experimental Results

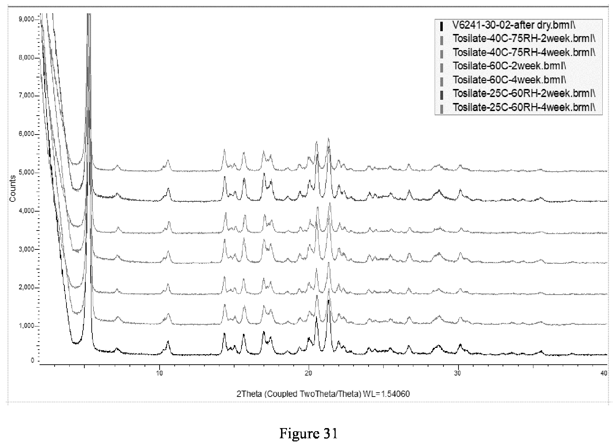

The physicochemical stability results of p-toluenesulfonate crystalline form A stored at 25°C/60% RH, 40°C/75% RH, and 60°C are shown in Table 16, and the comparison of XRPD patterns before and after the test is shown in Figure 31.

The results showed that p-toluenesulfonate crystalline form A exhibited good physicochemical stability after storage for 4 weeks at 25°C/60% RH, 40°C/75% RH, and 60°C.

**Table 16**

| Initial Crystalline Form | Storage Conditions | Storage Time | Appearance | Purity (%) | ee (%) | Crystalline Form Change |
|---|---|---|---|---|---|---|
| | - | Initial | Off-white powder | 99.81 | 100.00 | |
| | 40°C/75%RH (Open) | 2 weeks | Off-white powder | 99.84 | 100.00 | |
| p-toluenesulfo nate crystalline form A | | 4 weeks | Off-white powder | 99.80 | 100.00 | |
| | 60 °C (Open) | 2 weeks | Off-white powder | 99.66 | 100.00 | None |
| | | 4 weeks | Off-white powder | 99.50 | 100.00 | |
| | 25°C/60%R H (Open) | 2 weeks | Off-white powder | 99.83 | 100.00 | |
| | | 4 weeks | Off-white powder | 99.84 | 100.00 | |

High-temperature and high-humidity conditions arising from seasonal variation, regional climate differences, and environmental factors can affect the storage, transport, and manufacture of the API. p-Toluenesulfonate crystalline form A exhibited good physicochemical stability after storage for 4 weeks at 25°C/60% RH, 40°C/75% RH, and 60°C, helping to avoid impacts on drug quality during storage due to crystal transformation or purity loss.

The above examples are presented merely to illustrate the technical concepts and features of the present disclosure and to enable those skilled in the art to understand and implement the contents herein; they are not intended to limit the extent of protection of the present disclosure. All equivalent variations or modifications made in accordance with the spirit and scope of the present disclosure are intended to be encompassed within the extent of protection.

## Claims

1. A pharmaceutically acceptable salt, wherein the pharmaceutically acceptable salt formed by a compound of formula (I) with a pharmaceutically acceptable acid,

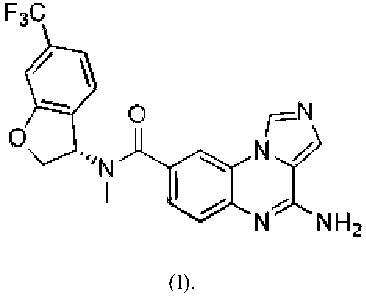

2. The pharmaceutically acceptable salt according to claim 1, wherein the molar ratio of the pharmaceutically acceptable acid to the compound of formula (I) is 1:2-2:1.

3. The pharmaceutically acceptable salt according to claim 1, wherein it is a crystalline solid.

4. The pharmaceutically acceptable salt according to claim 2, wherein the molar ratio of the pharmaceutically acceptable acid to the compound of formula (I) is 1:1.

5. The pharmaceutically acceptable salt according to claim 2, wherein the molar ratio of the pharmaceutically acceptable acid to the compound of formula (I) is 2:1.

6. The pharmaceutically acceptable salt according to claim 3, wherein the crystalline solid is an anhydrate.

7. The pharmaceutically acceptable salt according to claim 4, wherein the salt is selected from p-toluenesulfonate, maleate, benzenesulfonate, phosphate, or oxalate.

8. The pharmaceutically acceptable salt according to claim 5, wherein the salt is a diphenylsulfonate or a dihydrochloride.

9. The pharmaceutically acceptable salt according to claim 6 or claim 7, wherein the salt is a maleate.

10. The pharmaceutically acceptable salt according to claim 6 or claim 7, wherein the salt is a p-toluenesulfonate.

11. The pharmaceutically acceptable salt according to claim 6 or claim 7, wherein the salt is a benzenesulfonate.

12. The pharmaceutically acceptable salt according to claim 6 or claim 7, wherein the salt is a phosphate.

13. The pharmaceutically acceptable salt according to claim 6 or claim 7, wherein the salt is an oxalate.

14. The pharmaceutically acceptable salt according to claim 6 or claim 7, wherein the salt is a diphenylsulfonate.

15. The pharmaceutically acceptable salt according to claim 9, **characterized by** at least one of the following:
(i) using Cu-Kα radiation, the X-ray powder diffraction pattern comprises three or more peaks at 20 values selected from any of the following: 5.31°±0.2°, 14.63°±0.2°, 15.15°±0.2°, 17.30°±0.2°, 18.87°±0.2°, 21.42°±0.2°, 26.84°±0.2°, 27.63°±0.2°, and 30.56°±0.2°;
(ii) the X-ray powder diffraction pattern is basically as shown in Figure 3;
(iii) endothermic peak onset at 233.9°C±3°C;
(iv) after storage at 40°C and 75% RH for four weeks, an X-ray powder diffraction pattern basically the same as that of (i) or (ii) is observed;
(v) after storage at 60°C for four weeks, an X-ray powder diffraction pattern basically the same as that of (i) or (ii) is observed;
(vi) hygroscopic weight gain < 0.30% at 0-80% RH;
(vii) a single-crystal structure having unit cell parameters substantially equal to the following:
Crystal system and space group: triclinic, P1, $a = 8.73 \pm 0.5 Å$ $b = 8.78 \pm 0.5 Å$ $c = 16.89 \pm 0.5 Å$ $α = 90.17 ° \pm 1 °$ $β = 101.37 ° \pm 1 °$ $γ = 111.41 ° \pm 1 ° .$

16. The pharmaceutically acceptable salt according to claim 15, wherein when measured using Cu-Kα radiation, the X-ray powder diffraction pattern exhibits a characteristic peak at 20 = 5.31°±0.2° and two characteristic peaks at any two of 2θ = 17.30°±0.2°, 18.87°±0.2°, 21.42°±0.2°, 26.84°±0.2°, 27.63°±0.2°, and 30.56°±0.2°.

17. The pharmaceutically acceptable salt according to claim 15, wherein when measured using Cu-Kα radiation, the X-ray powder diffraction pattern exhibits characteristic peaks at 20 = 5.31°±0.2° and 18.87°±0.2°, and one additional characteristic peak at any one of 2θ = 17.30±0.2°, 21.42±0.2°, 26.84±0.2°, 27.63±0.2°, and 30.56±0.2°.

18. The pharmaceutically acceptable salt according to claim 15, wherein when measured using Cu-Kα radiation, the X-ray powder diffraction pattern exhibits characteristic peaks at 20 = 5.31°±0.2°, 17.30°±0.2°, 18.87°±0.2°, 21.42°±0.2°, and 27.63°±0.2°.

19. The pharmaceutically acceptable salt according to claim 10, **characterized by** at least one of the following:
(i) using Cu-Kα radiation, the X-ray powder diffraction pattern comprises three or more peaks at 20 values selected from any of the following: 5.42°±0.2°, 10.74°±0.2°, 17.15°±0.2°, 17.65°±0.2°, 20.12°±0.2°, 20.65°±0.2°, 21.47°±0.2°, and 30.29°±0.2°;
(ii) the X-ray powder diffraction pattern is basically as shown in Figure 17;
(iii) endothermic peak onset at 259.5°C±3°C;
(iv) after storage at 40°C and 75% RH for four weeks, an X-ray powder diffraction pattern basically the same as that of (i) or (ii) is observed;
(v) after storage at 60°C for four weeks, an X-ray powder diffraction pattern basically the same as that of (i) or (ii) is observed;
(vi) hygroscopic weight gain < 0.50% at 0-80% RH.

20. The pharmaceutically acceptable salt according to claim 19, wherein when measured using Cu-Kα radiation, the X-ray powder diffraction pattern exhibits characteristic peaks at 20 = 5.42°±0.2°, 10.74°±0.2°, and 21.47°±0.2°.

21. The pharmaceutically acceptable salt according to claim 11, **characterized by** at least one of the following:
(i) using Cu-Kα radiation, the X-ray powder diffraction pattern comprises three or more peaks at 20 values selected from any of the following: 5.48°±0.2°, 7.46°±0.2°, 14.93°±0.2°, 16.09°±0.2°, 17.66°±0.2°, and 21.61°±0.2°;
(ii) the X-ray powder diffraction pattern is basically as shown in Figure 13;
(iii) endothermic peak onset at 218.7°C±3°C.

22. The pharmaceutically acceptable salt according to claim 21, wherein the X-ray powder diffraction pattern exhibits characteristic peaks at 20 = 5.48±0.2°, 14.93±0.2°, and 21.61±0.2°.

23. The pharmaceutically acceptable salt according to claim 12, **characterized by** at least one of the following:
(i) using Cu-Kα radiation, the X-ray powder diffraction pattern comprises three or more peaks at 2θ values selected from any of the following: 6.26±0.2°, 11.66±0.2°, 12.54±0.2°, 13.62±0.2°, 15.95±0.2°, 21.65±0.2°, 22.32°±0.2°, and 23.42±0.2°;
(ii) the X-ray powder diffraction pattern is basically as shown in Figure 11.

24. The pharmaceutically acceptable salt according to claim 23, wherein the X-ray powder diffraction pattern exhibits characteristic peaks at 20 = 6.26±0.2°, 11.66±0.2°, and 15.95±0.2°.

25. The pharmaceutically acceptable salt according to claim 13, **characterized by** at least one of the following:
(i) using Cu-Kα radiation, the X-ray powder diffraction pattern comprises three or more peaks at 2θ values selected from any of the following: 5.20°±0.2°, 7.85°±0.2°, 10.46°±0.2°, 15.52°±0.2°, 16.51°±0.2°, 17.46°±0.2°, 18.29°±0.2°, 19.56°±0.2°, and 20.98°±0.2°;
(ii) the X-ray powder diffraction pattern is basically as shown in Figure 20.

26. The pharmaceutically acceptable salt according to claim 25, wherein the X-ray powder diffraction pattern exhibits characteristic peaks at 20 = 5.20±0.2°, 7.85±0.2°, and 10.46±0.2°.

27. The pharmaceutically acceptable salt according to claim 14, **characterized by** at least one of the following:
(i) using Cu-Kα radiation, the X-ray powder diffraction pattern comprises three or more peaks at 2θ values selected from any of the following: 4.66°±0.2°, 5.73°±0.2°, 9.23°±0.2°, 16.44°±0.2°, 17.27°±0.2°, 19.93°±0.2°, 20.64°±0.2°, and 21.77°±0.2°;
(ii) the X-ray powder diffraction pattern is basically as shown in Figure 15;
(vii) endothermic peak onset at 180.2°C±3°C.

28. The pharmaceutically acceptable salt according to claim 27, wherein when measured using Cu-Kα radiation, the X-ray powder diffraction pattern exhibits characteristic peaks at 20 = 4.66°±0.2°, 5.73°±0.2°, and 9.23°±0.2°.

29. A pharmaceutical composition comprising the pharmaceutically acceptable salt according to any one of claims 1-28.

30. Use of the pharmaceutically acceptable salt according to any one of claims 1-28 or the pharmaceutical composition according to claim 29 in the manufacture of a medicament for treating diseases amenable to treatment with PRMT5 inhibitors.
